# EUROPEAN PATENT SPECIFICATION

(11) **EP 4 344 654 B1**
(45) Date of publication and mention of the grant of the patent: **01.07.2026**
(21) Application number: 22198097.2
(22) Date of filing: 27.09.2022
(51) Int. Cl.: B06B 1/06, A61B 8/12, A61B 8/00

(54) **ULTRASOUND ENDOSCOPY**
ULTRASCHALLENDOSKOPIE
ENDOSCOPIE PAR ULTRASONS

(43) Date of publication of application: 03.04.2024
(73) Proprietor: Ambu A/S, 2750 Ballerup (DK)
(72) Inventor: DIEDERICHSEN, Søren Elmin, 2750 Ballerup (DK); SONNENBORG, Finn, 3600 Frederikssund (DK); SØRENSEN, Morten, 2750 Ballerup (DK)
(74) Representative: AWA Denmark A/S

(56) References cited:
- EP-A1- 1 498 071
- EP-A1- 3 437 565
- JP-A- H05 244 693
- JP-A- H07 274 295

## Description

### Field

The present disclosure relates to an endoscope, a printed flexible electrical circuit, and a system.

### Background

Endoscopes are widely used in hospitals for visually examining body cavities and obtaining samples of tissue identified as potentially pathological. An endoscope typically comprises an image capturing device arranged at the distal end of the endoscope either looking forward or to the side.

The image capturing device is however restricted by the limited penetration depth of visible light.

To examining deeper structures other imaging modalities must be used.

One possibility is to provide the endoscope with an ultrasound transducer. By using emission of ultrasound waves deeper structures may be examined.

A modern ultrasound transducer comprises a plurality of ultrasound transducer element such as 32 or more ultrasound transducer elements. Each ultrasound transducer element must be provided with an electrical conductor for providing and receiving electrical signals.

Performing the required electrical wiring is however a difficult and time-consuming task. This is one of the reasons why it is complex and expensive to manufacture a ultrasound transducer.

Ultrasound endoscopes are therefore significantly more expensive than regular endoscopes.

Availability of endoscopic ultrasound is therefore limited for some patient groups.

EP1498071 discloses an ultrasound probe including a transducer unit 1 having a plurality of channels arranged for transmitting and receiving an ultrasound wave, and flexible circuit board 2 connected to the respective channels of transducer unit 1 in which a signal line for supplying a transmission signal and extracting a reception signal to/from transducer unit 1 is provided, wherein flexible circuit board 2 forms at least two channel blocks made by dividing the plurality of channels and spirally wound individually.

Furthermore, endoscopes are difficult to clean and there exist the risk of cross-contamination, i.e. that a pathological condition is transferred between patients as result of improper cleaning.

The most secure solution has been the introduction of single-use endoscopes. A single use endoscope eliminates any risk of cross-contamination.

The cost of an ultrasound transducer has however made it difficult to provide single use ultrasound endoscopes. This is problematic since the provision of an ultrasound probe at the distal end of the endoscope makes the endoscope even harder to clean.

Thus, it remains a problem to provide an improved ultrasound transducer.

### Summary

According to a first aspect, the present disclosure relates to an endoscope comprising a handle, an insertion tube having a proximal end and a distal end, a first flexible cable and an ultrasound transducer for receiving and transmitting ultrasound, the ultrasound transducer comprising an ultrasound transducer array attached to the distal end of the insertion tube, the handle being attached to the proximal end of the insertion tube, the first flexible cable is extending from the handle and being electrically connected to the ultrasound transducer array and is connectable to an ultrasound processing apparatus and configured to provide the ultrasound processing apparatus with electrical signals generate by the ultrasound transducer array and receive electrical signals generated by the ultrasound processing apparatus for the ultrasound transducer array, and wherein the ultrasound transducer array comprises a plurality of ultrasound transducer elements, and wherein the ultrasound transducer comprises an electrical circuit for receiving and providing electrical signals to the plurality of ultrasound transducer elements, and a support structure having an upper surface facing the plurality of ultrasound transducer elements and extending in a direction parallel to a first axis, wherein the electrical circuit comprises
a flexible base section comprising a plurality of first electrical contacts each first electrical contact being connected to an ultrasound transducer element for providing and receiving electrical signals to / from the ultrasound transducer element and extending along a central axis being parallel to the first axis, the flexible base section being arranged on the upper surface of the support structure,
a plurality of first flexible electrical conductors each first flexible electrical conductor being electrically connected to a first electrical contact,
a contact section comprising a plurality of second electrical contacts each second electrical contact being electrically connected to a first electrical conductor and further electrically connected or connectable to a second electrical conductor or a transducer port of an ultrasound processing apparatus, and
wherein the flexible base section and the plurality of first flexible electrical conductors is a single flexible printed circuit and wherein the first flexible electrical conductors extend from the flexible base section and into the handle.

Consequently, by using an electrical circuit provided with a flexible base section having a plurality of electrical contacts and where a plurality of first flexible electrical conductors extend into the handle, the plurality of ultrasound transducer elements may be provided with an electrical connection in a single process step. This makes it simple to manufacture.

The flexible printed circuit may be made using Photolithography. An adhesive may be provided between the lower side of the ultrasound transducer array and the flexible base section. Correspondingly, an adhesive may be provided between the flexible base section and the upper surface of the support structure. After attachment to the plurality of ultrasound transduce elements the flexibility of the flexible base section is limited by the flexibility of the ultrasound transducer array.

The ultrasound transducer elements may be made from a piezo electric block. The piezoelectric block may be made of any kind of piezoelectric material such as lead zirconate titanate (PZT), Quartz, Barium titanate, Poly(vinylidene fluoride) (PVDF), or a piezoelectric composite material.

The ultrasound transducer may be provided with one or more acoustic matching layers configured to provide an acoustic impedance gradient between the acoustic impedance of the tissue and the acoustic impedance of the ultrasound transducer elements. The acoustic matching layer may be made of epoxy, polyurethane, or polystyrene.

The ultrasound transducer may be configured to generate ultrasound with a centre frequency between 1 MHz and 15 MHz, or 2MHz and 8MHz.

The plurality of ultrasound transducer elements may be arranged in a common reference plane and configured to image a single image plane.

The plurality of ultrasound transducer elements may have been pre-poled and metalized before being attached to flexible base section. As an example, one or two layers of a nickel-chromium alloy may be applied to the plurality of ultrasound transducer elements. Alternatively / additionally 1 or two layers of Au may be applied. The layer may preferably be thin layers having a thickness of 10-20nm. The layers may improve the piezoelectric properties of the piezoelectric block.

The support structure is preferably made of a material configured to absorb ultrasound energy. The material may be a composite material. As an example, the material may be an epoxy mixed with tungsten powder.

The upper surface of the support structure may be a convex upper surface, i.e. the ultrasound transducer array may be a curvilinear ultrasound transducer array. Alternatively, the upper surface of the support structure may be planar, i.e. the ultrasound transducer array may be a linear array.

The ultrasound transducer may further comprise an acoustic lens for focusing the ultrasound energy. The acoustic lens may be configured to focus the ultrasound energy in the image plane.

In some embodiments, the plurality of first flexible electrical conductors for a first part of their length extends inside the insertion tube, for a second part of their length extends inside the handle, and for a third part of their length extend inside the first flexible cable.

In some embodiments, the first flexible cable comprises an ultrasound connector insertable into a transducer port of an ultrasound processing apparatus for providing electrical signals generated by the ultrasound transducer array to the ultrasound processing apparatus and receiving electrical signals generated by the ultrasound processing apparatus, wherein the plurality of first flexible electrical conductors extend throughout the entire length of the first flexible cable and the ultrasound connector is the contact section.

Consequently, by using a single flexible printed circuit for the entire electrical connection between the ultrasound array and the ultrasound connector it becomes simpler to manufacture the endoscope.

In some embodiments, the endoscope further comprises an image capturing device and wherein the first flexible cable is further configured to receive electrical signals from the image capturing device.

Consequently, by using a single flexible cable to transfer signals from both the ultrasound transducer and the image capturing device it may become simpler for a user to operate the endoscope.

In some embodiments, the plurality of first flexible electrical conductors are folded at least once to extend their length.

Consequently, the cost of manufacturing the flexible printed circuit may be reduced.

In some embodiments, the upper surface is a convex upper surface, the plurality of first flexible electrical conductors are bent around the support structure at a first curve section and at a second curve section, the first curve section being separate from the second curve section.

Consequently, by providing the electrical circuit with a plurality of flexible electrical conductors bent around the support structure at a first curve section and second curve section, a single electrical circuit may be used to transport the electrical signal to and from the plurality of electrical contacts as the electric circuit may conform to the curvature of the convex upper surface.

The plurality of first flexible electrical conductors may be bent around the support structure at more curve sections than the first curve section and the second curve section. As an example, the plurality of first flexible electrical conductors may be bend around the support structure at least at three, four, six, or eight curve sections.

In some embodiments, the support structure has a first side surface arranged in a plane being perpendicular to the first axis, the first side surface being connected to the convex upper surface and wherein the first curve section is arranged along the connection between the convex upper surface and the first side surface.

In some embodiments, the second curve section is arranged along the connection between the convex upper surface and the first side surface and divided from the first curve section by a gap.

Consequently, it may become possible to guide a significant number (or all) of the flexible electrical conductors away from the convex upper surface of the support structure in a simple and compact manner.

In some embodiments, the support structure comprises a concave lower surface opposite to the convex upper surface, the first side surface is connected to the concave lower surface, the gap extends along the entire first side of the support structure, and wherein the plurality of the first flexible electrical conductors are further bent around the support structure at a third curve section and at a fourth curve section, the third curve section being separate from the fourth curve section by the gap, and wherein the third curve section and the fourth curve section are arranged along the connection between the first side surface and the concave lower surface.

Consequently, the concave lower surface may be utilized to provide space for directing the plurality of first flexible electrical conductors away from the ultrasound transducer elements. This may reduce the amount of space required by the electric circuit.

According to a second aspect, the present disclosure relates to a printed flexible electrical circuit for receiving and providing electrical signals to an ultrasound transducer array comprising a plurality of ultrasound transducer elements, the printed flexible electrical circuit comprising,
a flexible base section comprising a plurality of first electrical contacts each first electrical contact being connectable to an ultrasound transducer element for providing and receiving electrical signals to the ultrasound transducer element and extending along a central axis being parallel to a first axis,
a plurality of first flexible electrical conductors each first flexible electrical conductor being electrically connected to a first electrical contact, and
wherein the plurality of first flexible electrical conductors are connectable to an ultrasound connector whereby the first flexible electrical conductors may be responsible for the entire electrical coupling between the ultrasound connector and the ultrasound transducer array.

In some embodiments, the plurality of first flexible electrical conductors have a length of at least 40 cm.

According to a third aspect, the present disclosure relates to a system comprising an endoscope as disclosed in relation to the first aspect of the disclosure and an ultrasound processing apparatus, wherein the endoscope comprises an image capturing device and the first flexible cable is connectable to the ultrasound processing apparatus.

Ultrasound processing apparatuses are well-known in the art. Ultrasound processing apparatuses are configured to generate the electrical signals needed by the ultrasound transducer elements to generate the ultrasound waves. The electrical signal should have a centre frequency substantially matching the centre frequency of the ultrasound transducer elements. Furthermore, the signal should have a length and amplitude securing that a sufficient SNR is achieved. Furthermore, the ultrasound processing apparatus is configured to generate the ultrasound images by processing the signals generated by the ultrasound transducer elements. The ultrasound imaging may be done by using ultrasound beamforming techniques such as delay and sum beamforming in receive. In transmit, the ultrasound energy may be focused in a particular point by applying a suitable delay to the individual ultrasound transducer elements. Alternatively, a plane wave or a diverging ultrasound wave may be used in transmit.

The ultrasound processing apparatus may be adapted to cause a display to display a live representation of the electrical signals received from the ultrasound transducer. The ultrasound processing apparatus may be couplable to a display. The ultrasound processing apparatus may comprise a display for displaying a live representation of the electrical signals received from the ultrasound transducer and/or the image capturing device.

The ultrasound processing apparatus may be a portable ultrasound processing apparatus.

In some embodiments, the first flexible cable is further configured to receive electrical signals from the image capturing device and provide the received electrical signals to the ultrasound processing apparatus, and wherein the ultrasound processing apparatus comprise one or more processing units configured to process both the signals received from the ultrasound transducer and the signals received from the image capturing device.

Consequently, by using a single flexible cable to transfer signals from both the ultrasound transducer and the image capturing device it may becomes simpler for user to operate the endoscope.

The ultrasound processing apparatus may comprise one or more processing units configured to process both the signals received from the ultrasound transducer and the signals received from the image capturing device.

Alternatively, the ultrasound processing apparatus may comprise a first set of one or more processing units configured to process the signals received from the ultrasound transducer and a second set of one or more processing units configured to process the signals received form the image capturing device. The first set and second set of one or more processing units may be provided in the same housing or in separate housings where the separate housings are communicatively coupled.

In some embodiments, the system further comprises an video processing apparatus and wherein the endoscope further comprises a second flexible cable configured to receive electrical signals from the image capturing device and provide the received electrical signals to the video processing apparatus.

Consequently, it may become simpler to connect the endoscope to a separate ultrasound processing apparatus and video processing apparatus.

The video processing apparatus may be adapted to cause a display to display a live representation of the electrical signals received from image capturing device. The video processing apparatus may be couplable to a display. The video processing apparatus may comprise a display for displaying a live representation of the electrical signals received from the image capturing device.

The video processing apparatus may be a portable video processing apparatus.

In some embodiments, the first flexible cable and the second flexible cable are connected along a first part of their length and disconnected along a second part of their length.

Consequently, it may both become simpler for the user to operate the endoscope and connect the cables to a separate ultrasound processing apparatus and video processing apparatus.

The first flexible cable may be attached to the second cable using an adhesive. Additionally / alternatively an outer cable may be arranged around the first flexible cable and the second flexible cable along the first part of their length.

The different aspects of the present disclosure can be implemented in different ways including as an endoscope, a printed flexible electrical circuit, and an endoscope system described above and in the following, each yielding one or more of the benefits and advantages described in connection with at least one of the aspects described above, and each having one or more preferred embodiments corresponding to the preferred embodiments described in connection with at least one of the aspects described above and/or disclosed in the dependent claims. Furthermore, it will be appreciated that embodiments described in connection with one of the aspects described herein may equally be applied to the other aspects.

### Brief description of the drawings

The above and/or additional objects, features and advantages of the present disclosure, will be further elucidated by the following illustrative and non-limiting detailed description of embodiments of the present disclosure, with reference to the appended drawings, wherein:
Fig. 1a-b show schematically an endoscope according to an embodiment of the disclosure.
Fig. 2a-b show schematically a support structure for an ultrasound transducer according to an embodiment of the disclosure.
Fig. 3a-c show schematically a support structure and an electrical circuit for an ultrasound transducer according to an embodiment of the disclosure.
Fig. 4a-c show schematically a support structure and an electrical circuit for an ultrasound transducer according to an embodiment of the disclosure.
Fig. 5a-b show schematically a support structure and an electrical circuit for an ultrasound transducer according to an embodiment of the disclosure.
Fig. 6 shows schematically an ultrasound transducer according to an embodiment of the disclosure.
Fig. 7 shows schematically a system according to an embodiment of the disclosure.
Fig. 8 shows schematically a system according to an embodiment of the disclosure.

### Detailed description

In the following description, reference is made to the accompanying figures, which show by way of illustration how the embodiments of the present disclosure may be practiced.

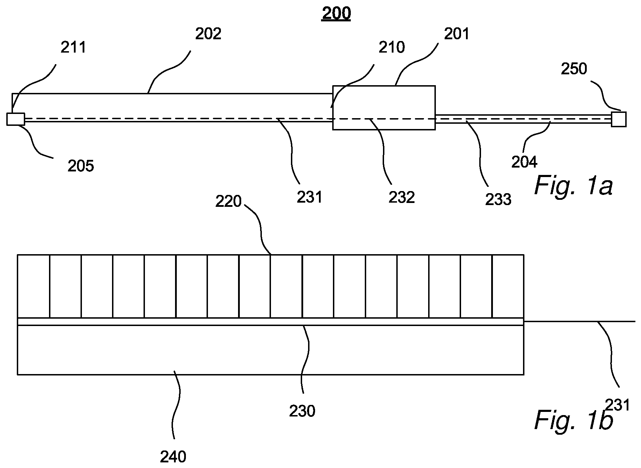

Fig. 1a-b show schematically an endoscope provided with an ultrasound transducer according to an embodiment of the present disclosure. Fig. 1a shows a view of the endoscope, and Fig. 1b shows a close-up of a part of the ultrasound transducer. The endoscope (200) comprises a handle (201), an insertion tube (202) having a proximal end (210) and a distal end (211), a first flexible cable (204) and an ultrasound transducer (205) for receiving and transmitting ultrasound. The ultrasound transducer (205) comprising an ultrasound transducer array (220) attached to the distal end (211) of the insertion tube (202), the handle (201) being attached to the proximal end (210) of the insertion tube (202), the first flexible cable (204) is extending from the handle (201) and being electrically connected to the ultrasound transducer array (220) and is connectable to an ultrasound processing apparatus (not shown) and configured to provide the ultrasound processing apparatus with electrical signals generate by the ultrasound transducer array (220) and receive electrical signals generated by the ultrasound processing apparatus for the ultrasound transducer array. The ultrasound transducer array (220) comprises a plurality of ultrasound transducer elements, an electrical circuit (230)-(233) for receiving and providing electrical signals to the plurality of ultrasound transducer elements, and a support structure (240) having an upper surface facing the plurality of ultrasound transducer elements and extending in a direction parallel to a first axis (not shown). The electrical circuit (230-233) comprises a flexible base section (230), a plurality of first flexible electrical conductors (231-233), and a contact section (250). The individual first flexible electrical conductors are not shown. The flexible base section (230) comprises a plurality of first electrical contacts (not shown) each first electrical contact being connected to an ultrasound transducer element for providing and receiving electrical signals to / from the ultrasound transducer element and extending along a central axis (not shown) being parallel to the first axis, the flexible base section (230) being arranged on the upper surface of the support structure (240). Each of the plurality of first flexible electrical conductors (231-233) being electrically connected to a first electrical contact. The contact section (250) comprising a plurality of second electrical contacts each second electrical contact being electrically connected to a first electrical conductor (230-233) and further electrically connected or connectable to a second electrical conductor or a transducer port of an ultrasound processing apparatus. The flexible base section (230) and the plurality of first flexible electrical conductors (231-233) is a single flexible printed circuit and the first flexible electrical conductors (231-233) extend from the flexible base section (230) and into the handle (201). In this embodiment, the plurality of first flexible electrical conductors (231-233) for a first part of their length (231) extends inside the insertion tube (202), for a second part of their length (232) extends inside the handle (201), and for a third part of their length (233) extend inside the first flexible cable.

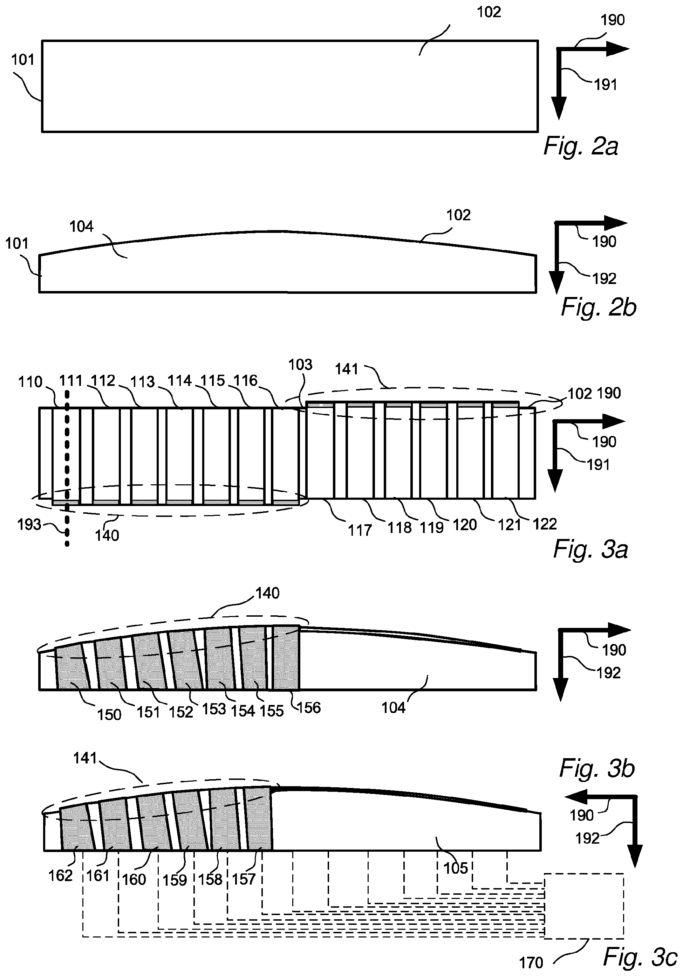

Fig. 2a-b show schematically a support structure (101) for an ultrasound transducer according to an embodiment of the disclosure, where Fig. 2a shows a top view and Fig. 2b shows a side view. The support structure (101) has a convex upper surface (102) for facing a plurality of ultrasound transducer elements. The convex upper surface (102) extends in a direction parallel to a first axis (191). There is further shown a second axis (190) and a third axis (192). The second axis (190) being perpendicular to the first axis (191), and the third axis (192) being perpendicular to both the first axis (191) and the second axis (190). In addition to supporting the ultrasound transducer elements, the support structure may assist in absorbing ultrasound energy. Thus, the support structure (101) is preferably made of a material configured to absorb ultrasound energy. The material may be a composite material. The material may be an epoxy mixed with tungsten powder.

Fig. 3a-c show schematically a support structure (101) and an electrical circuit for an ultrasound transducer according to an embodiment of the disclosure, where Fig. 3a shows a top view, Fig. 3b shows a first side view, and Fig. 3c shows a second side view. The support structure (101) corresponds to the support structure (101) shown in Fig. 2a-b. The electrical circuit comprises a flexible base section (103) comprising a plurality of first electrical contacts (110-122) each first electrical contact (110-122) being connectable to an ultrasound transducer element for providing and receiving electrical signals to / from the ultrasound transducer element. Each of the first electrical contacts (110-122) extends along a central axis (193) being parallel to the first axis (191). For simplicity only the central axis (193) for the first electrical contact 110 is shown. The flexible base section (103) is arranged on the convex upper surface (102) of the support structure (101). The first electrical contacts (110-122) should be made of a material having a high electrical conductivity such as Cu, Al, Ag, or Au. The area of the flexible base section (103) between the plurality of first electrical contacts (110-122) should be electrically isolating to prevent crosstalk between the ultrasound transducer elements. Correspondingly, the flexible base section (103) is preferably provided with one or more electrically isolating lower layers. For simplicity only 13 first electrical contacts (110-122) are shown. However, in other embodiments there may be at least 16, 32 or 64 first electrical contacts. The electrical circuit further comprises a plurality of first flexible electrical conductors (150-162) each first flexible electrical conductor being electrically connected to a first electrical contact (110-122). The plurality of first flexible electrical conductors (150-162) are responsible for transferring electrical signals from the plurality of first electrical contacts (110-122) towards an ultrasound processing apparatus and transferring electrical signals from an ultrasound processing apparatus towards the plurality of first electrical contacts (110-122). The plurality of first flexible electrical conductors (150-162) should be made of a material having a high electrical conductivity such as Cu, Al, Ag, or Au. The area between the plurality of first flexible electrical conductors (150-162) should be electrically isolating to prevent cross-talk between the ultrasound transducer elements. Correspondingly, the plurality of first flexible electrical conductors (150-162) are preferably provided with one or more electrically isolating upper layers and one or more electrically isolating lower layers. The plurality of first flexible electrical conductors (150-162) are bent around the support structure (101) at a first curve section (140) and at a second curve section (141), the first curve section (140) being separate from the second curve section (141). The plurality of first flexible electrical conductors comprises a first group of flexible electrical conductors (150-156) and a second group of flexible electrical conductors (157-162). The support structure (101) has a first side surface (104) being arranged in a plane (not shown) being perpendicular to the first axis (191), the first side surface (104) being connected to the convex upper surface (102) and wherein the first curve section (140) is arranged along the connection between the convex upper surface (102) and the first side surface (104). The support structure (101) has a second side surface (105) opposite to the first side surface (104), the second side surface (105) being arranged in a plane (not shown) being perpendicular to the first axis (191), the second side surface (105) being connected to the convex upper surface (102), the second curve section (141) is arranged along the connection between the convex upper surface (102) and the second side surface (105). The first group of flexible electrical conductors (150-156) are bent around the first curve section (140) and the second group of flexible electrical conductors (157-162) are bent around the second curve section (141). Only the first part of the plurality of first electrical conductors is shown in Fig. 3a-b. In Fig. 3c are the remaining part of the first electrical conductors schematically illustrated by dashed lines. The electrical circuit further comprises a contact section (170) comprising a plurality of second electrical contacts each second electrical contact being electrically connected to a first electrical conductor (150-162) and further electrical connected or connectable to a second electrical conductor or a transducer port of an ultrasound processing apparatus.

In some embodiments, the contact section (170) is the ultrasound connector that is inserted into the transducer port of an ultrasound processing apparatus and the first electrical conductors (150-162) are responsible for the entire electrical coupling between the plurality of first electrical contacts (110-122) and the ultrasound connector.

In other embodiments, each of the plurality of second electrical contacts of the contact section (170) are connected to a second electrical conductor and the first electrical conductors (150-162) are only responsible for part of the electrical coupling between the plurality of first electrical contacts (110-122) and the ultrasound connector. The second electrical conductor may be a coaxial cable or a printed flexible electrical conductor. In one embodiment, the contact section is located in the handle of the endoscope and the second electrical conductor extends into the first flexible cable.

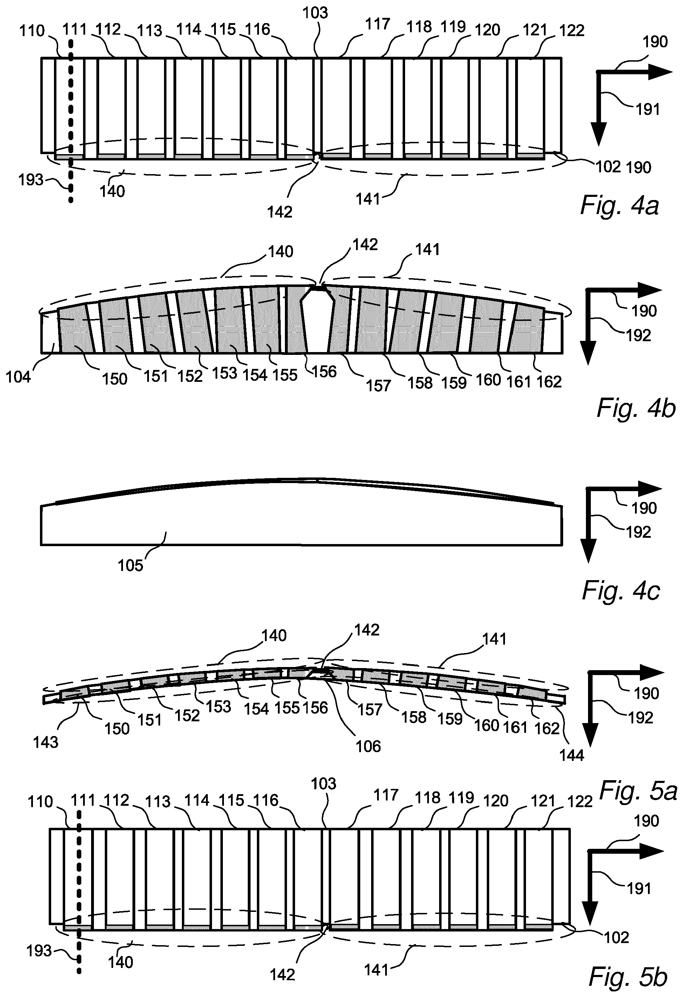

Fig. 4a-c show schematically a support structure (101) and an electrical circuit for an ultrasound transducer according to an embodiment of the disclosure, where Fig. 4a shows a top view, Fig. 4b shows a first side view, and Fig. 4c shows a second side view. The support structure (101) corresponds to the support structure (101) shown in Fig. 2a-b. The electrical circuit comprises a flexible base section (103) comprising a plurality of first electrical contacts (110-122) each first electrical contact (110-122) being connectable to an ultrasound transducer element for providing and receiving electrical signals to / from the ultrasound transducer element. Each of the first electrical contacts (110-122) extends along a central axis (193) being parallel to the first axis (191). For simplicity only the central axis (193) for the first electrical contact 110 is shown. The flexible base section (103) is arranged on the convex upper surface (102) of the support structure (101). The electrical circuit further comprises a plurality of first flexible electrical conductors (150-162) each first flexible electrical conductor being electrically connected to a first electrical contact (110-122). The plurality of first flexible electrical conductors (150-162) are responsible for transferring electrical signals from the plurality of first electrical contacts (110-122) towards an ultrasound processing apparatus and transferring electrical signals from an ultrasound processing apparatus towards the plurality of first electrical contacts (110-122). The support structure (101) has a first side surface (104) arranged in a plane (not shown) being perpendicular to the first axis (191), the first side surface (104) being connected to the convex upper surface (102). The plurality of first flexible electrical conductors (150-162) are bent around the support structure (101) at a first curve section (140) and at a second curve section (141), the first curve section (140) being separate from the second curve section (141). The first curve section (140) and the second curve section (141) are arranged along the connection between the convex upper surface (102) and the first side surface (104) and divided by a gap (142). Only the first part of the plurality of first electrical conductors is shown in Fig. 4a-c. The electrical circuit may further comprise a contact section (170) comprising a plurality of second electrical contacts each second electrical contact being electrically connected to a first electrical conductor (150-162) and further electrical connected or connectable to a second electrical conductor or a transducer port of an ultrasound system.

Fig. 5a-b show schematically a support structure (101) and an electrical circuit for an ultrasound transducer according to an embodiment of the disclosure, where Fig. 5a shows a top view and Fig. 5b shows a side view. The embodiment shown in Fig. 5a-b corresponds to the embodiment shown in Fig. 4a-c with the difference that the support structure (101) comprises a concave lower surface (106) opposite to the convex upper surface (102), the first side surface (104) is connected to the concave lower surface (106) and the gap (142) extends along the entire first side (104) of the support structure (101). The plurality of first flexible electrical conductors (150-162) are further bent around the support structure at a third curve section (143) and at a fourth curve section (144), the third curve section (143) being separate from the fourth curve section (144) by the gap (142), and wherein the third curve section (143) and the fourth curve section (144) are arranged along the connection between the first side surface (104) and the concave lower surface (106). Consequently, the concave lower surface may be utilized to provide space for directing the plurality of first flexible electrical conductors away from the ultrasound transducer elements. This may reduce the amount of space required by the electric circuit.

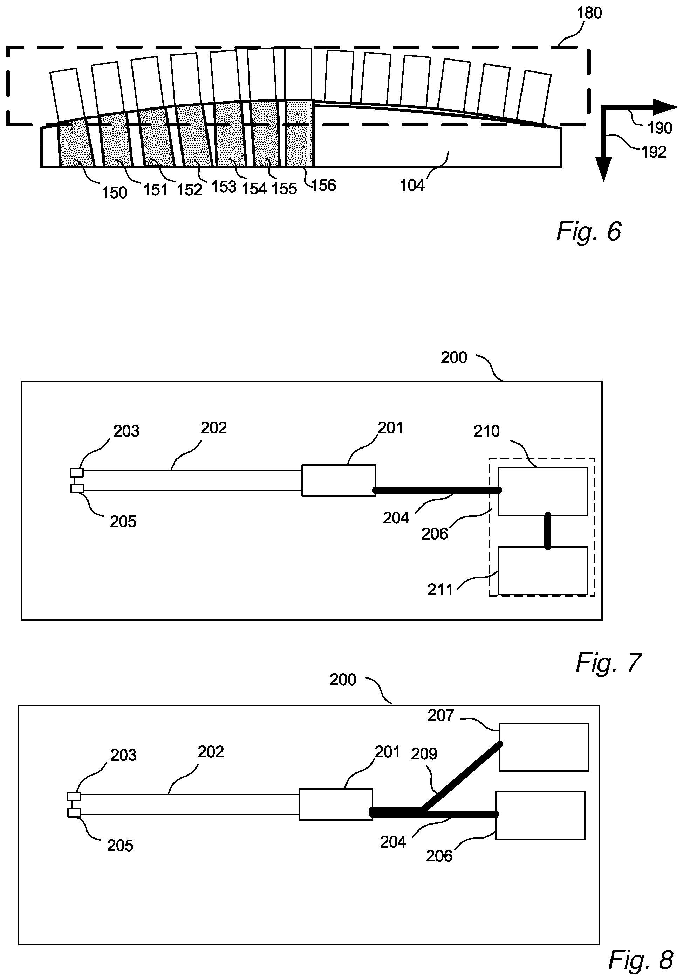

Fig. 6 shows schematically an ultrasound transducer according to an embodiment of the disclosure. The ultrasound transducer comprises a support structure and an electrical circuit corresponding to the support structure and the electrical circuit disclosed in relation to Fig. 3a-c. Furthermore, the ultrasound transducer comprises a convex ultrasound transducer array comprising a plurality of ultrasound transducer elements (180), where each first electrical contact (110-122) is connected to an ultrasound transducer element for providing and receiving electrical signals to / from the ultrasound transducer element.

Fig. 7 shows schematically a system (200) comprising an endoscope and an ultrasound processing apparatus (206) according to an embodiment of the disclosure. The endoscope comprises a handle (201), an insertion tube (202), an image capturing device (203), a first flexible cable (204) and an ultrasound transducer (205). The ultrasound transducer may be an ultrasound transducer as disclosed in Figs.1-6. The handle (201) is attached to a proximal end of the insertion tube (202) and the image capturing device (203) and the ultrasound transducer (205) are attached to a distal end of the insertion tube (202). The first flexible cable (204) is extending from the handle (201) and is electrically connected to the ultrasound transducer (205) and wherein the first flexible cable is connected to the ultrasound processing apparatus (206) and configured to provide the ultrasound processing apparatus (206) with electrical signals generate by the ultrasound transducer (205) and receive electrical signals generated by the ultrasound processing apparatus (206) for the ultrasound transducer (205). In this embodiment, the first flexible cable (204) is further configured to receive electrical signals from the image capturing device (203) and provide the received electrical signals to the ultrasound processing apparatus (206). The ultrasound processing apparatus (206) comprises a first processing unit (210) configured to process electrical signals generate by the ultrasound transducer (205) and a second processing unit (211) configured to process image signals recorded by the image capturing device (203).

Fig. 8 shows schematically a system (200) comprising an endoscope an ultrasound processing apparatus (206), and an imaging unit (207) according to an embodiment of the disclosure. The endoscope comprises a handle (201), an insertion tube (202), an image capturing device (203), a first flexible cable (204), a second flexible cable (209) and an ultrasound transducer (205). The ultrasound transducer (205) may be an ultrasound transducer as disclosed in Figs.1-6. The handle (201) is attached to a proximal end of the insertion tube (202) and the image capturing device (203) and the ultrasound transducer (205) are attached to a distal end of the insertion tube (202). The first flexible cable (204) is extending from the handle (201) and is electrically connected to a plurality of ultrasound transducer elements via an electrical circuit and wherein the first flexible cable is connected to the ultrasound processing apparatus (206) and configured to provide the ultrasound processing apparatus (206) with electrical signals generate by the ultrasound transducer (205) and receive electrical signals generated by the ultrasound processing apparatus (206) for the ultrasound transducer (205). In this embodiment, the second flexible cable (209) is configured to receive electrical signals from the image capturing device (203) and provide the received electrical signals to the imaging unit (209).

Although some embodiments have been described and shown in detail, the invention is not restricted to them, but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. An endoscope comprising a handle, an insertion tube having a proximal end and a distal end, a first flexible cable and an ultrasound transducer for receiving and transmitting ultrasound, the ultrasound transducer comprising an ultrasound transducer array attached to the distal end of the insertion tube, the handle being attached to the proximal end of the insertion tube, the first flexible cable is extending from the handle and being electrically connected to the ultrasound transducer array and is connectable to an ultrasound processing apparatus and configured to provide the ultrasound processing apparatus with electrical signals generated by the ultrasound transducer array and receive electrical signals generated by the ultrasound processing apparatus for the ultrasound transducer array, and wherein the ultrasound transducer array comprises a plurality of ultrasound transducer elements (180), and wherein the ultrasound transducer comprises an electrical circuit for receiving and providing electrical signals to the plurality of ultrasound transducer elements (180), and a support structure (101) having an upper surface (102) facing the plurality of ultrasound transducer elements (180) and extending in a direction parallel to a first axis (191), wherein the electrical circuit comprises
a flexible base section (103) comprising a plurality of first electrical contacts (110-122) each first electrical contact (110-122) being connected to an ultrasound transducer element for providing and receiving electrical signals to / from the ultrasound transducer element and extending along a central axis (193) being parallel to the first axis (191), the flexible base section (103) being arranged on the upper surface (102) of the support structure (101),
a plurality of first flexible electrical conductors (150-162) each first flexible electrical conductor being electrically connected to a first electrical contact (110-122),
a contact section comprising a plurality of second electrical contacts each second electrical contact being electrically connected to a first electrical conductor (150-162) and further electrically connected or connectable to a second electrical conductor or a transducer port of an ultrasound processing apparatus, and
**characterized in that** the flexible base section and the plurality of first flexible electrical conductors is a single flexible printed circuit and wherein the first flexible electrical conductors (150-162) extend from the flexible base section (103) and into the handle.

2. An endoscope according to claim 1, wherein the plurality of first flexible electrical conductors (150-162) for a first part of their length extends inside the insertion tube, for a second part of their length extends inside the handle, and for a third part of their length extend inside the first flexible cable.

3. An endoscope according to claim 2, wherein the first flexible cable comprises an ultrasound connector insertable into a transducer port of an ultrasound processing apparatus for providing electrical signals generated by the ultrasound transducer array to the ultrasound processing apparatus and receiving electrical signals generated by the ultrasound processing apparatus, wherein the plurality of first flexible electrical conductors (150-162) extend throughout the entire length of the first flexible cable and the ultrasound connector is the contact section.

4. An endoscope according to claim 3, wherein the endoscope further comprises an image capturing device and wherein the first flexible cable is further configured to receive electrical signals from the image capturing device.

5. An endoscope according to any one of claims 1 to 4, wherein the plurality of first flexible electrical conductors (150-162) are folded at least once to extend their length.

6. An endoscope according to any one of claims 1 to 5, wherein the upper surface (102) is a convex upper surface, the plurality of first flexible electrical conductors (150-162) are bent around the support structure (101) at a first curve section (140) and at a second curve section (141), the first curve section (140) being separate from the second curve section (141).

7. An endoscope according to claim 6, wherein the support structure (101) has a first side surface (104) arranged in a plane being perpendicular to the first axis (191), the first side surface (104) being connected to the convex upper surface (102) and wherein the first curve section (140) is arranged along the connection between the convex upper surface (102) and the first side surface (104).

8. An endoscope according to claim 7, wherein the second curve section (141) is arranged along the connection between the convex upper surface and the first side surface (104) and divided from the first curve section (140) by a gap (142).

9. An endoscope according to claim 8, wherein the support structure comprises a concave lower surface (106) opposite to the convex upper surface (102), the first side surface (104) is connected to the concave lower surface (106), the gap (142) extends along the entire first side (104) of the support structure (101), and wherein the plurality of the first flexible electrical conductors (150-162) are further bent around the support structure at a third curve section (143) and at a fourth curve section (144), the third curve section (143) being separate from the fourth curve section (144) by the gap (142), and wherein the third curve section (143) and the fourth curve section (144) are arranged along the connection between the first side surface (104) and the concave lower surface (106).

10. A printed flexible electrical circuit for receiving and providing electrical signals to an ultrasound transducer array comprising a plurality of ultrasound transducer elements, the printed flexible electrical circuit forming part of an endoscope according to claim 1 and comprising
a flexible base section (103) comprising a plurality of first electrical contacts (110-122) each first electrical contact (110-122) being connectable to an ultrasound transducer element for providing and receiving electrical signals to the ultrasound transducer element and extending along a central axis (193) being parallel to a first axis (191),
a plurality of first flexible electrical conductors (150-162) each first flexible electrical conductor being electrically connected to a first electrical contact (110-122), and
wherein the plurality of first flexible electrical conductors (150-162) are connectable to an ultrasound connector whereby the first flexible electrical conductors may be responsible for the entire electrical coupling between the ultrasound connector and the ultrasound transducer array.

11. A printed flexible electrical circuit wherein the plurality of first flexible electrical conductors (150-162) have a length of at least 40 cm.

12. A system comprising an endoscope according to any one of claims 1 to 9 and an ultrasound processing apparatus, wherein the endoscope comprises an image capturing device and the first flexible cable is connectable to the ultrasound processing apparatus.

13. A system according to claim 12, wherein the first flexible cable is further configured to receive electrical signals from the image capturing device and provide the received electrical signals to the ultrasound processing apparatus, and wherein the ultrasound processing apparatus comprise one or more processing units configured to process both the signals received from the ultrasound transducer and the signals received from the image capturing device.

14. A system according to claim 13, wherein the system further comprises an video processing apparatus and wherein the endoscope further comprises a second flexible cable configured to receive electrical signals from the image capturing device and provide the received electrical signals to the video processing apparatus.

## Patentansprüche

1. Endoskop, umfassend einen Griff, ein Einführrohr mit einem proximalen Ende und einem distalen Ende, ein erstes flexibles Kabel und einen Ultraschallwandler zum Empfangen und Senden von Ultraschall, wobei der Ultraschallwandler ein Ultraschallwandler-Array umfasst, das an dem distalen Ende des Einführrohrs angebracht ist, wobei der Griff an dem proximalen Ende des Einführrohrs angebracht ist, das erste flexible Kabel sich von dem Griff erstreckt und elektrisch mit dem Ultraschallwandler-Array verbunden ist und mit einem Ultraschallverarbeitungsgerät verbindbar ist und dazu ausgelegt ist, dem Ultraschallverarbeitungsgerät elektrische Signale bereitzustellen, die durch das Ultraschallwandler-Array generiert werden, und elektrische Signale zu empfangen, die durch das Ultraschallverarbeitungsgerät für das Ultraschallwandler-Array generiert werden, und wobei das Ultraschallwandler-Array eine Vielzahl von Ultraschallwandlerelementen (180) umfasst, und wobei der Ultraschallwandler eine elektrische Schaltung zum Empfangen und Bereitstellen elektrischer Signale an die Vielzahl von Ultraschallwandlerelementen (180) und eine Stützstruktur (101) mit einer oberen Oberfläche (102), die der Vielzahl von Ultraschallwandlerelementen (180) zugewandt ist und sich in einer Richtung parallel zu einer ersten Achse (191) erstreckt, umfasst, wobei die elektrische Schaltung umfasst
einen flexiblen Basisabschnitt (103), der eine Vielzahl von ersten elektrischen Kontakten (110-122) umfasst, wobei jeder erste elektrische Kontakt (110-122) mit einem Ultraschallwandlerelement zum Bereitstellen und Empfangen elektrischer Signale zu / von dem Ultraschallwandlerelement verbunden ist und sich entlang einer Mittelachse (193) erstreckt, die parallel zu der ersten Achse (191) ist, wobei der flexible Basisabschnitt (103) auf der oberen Oberfläche (102) der Stützstruktur (101) angeordnet ist,
eine Vielzahl von ersten flexiblen elektrischen Leitern (150-162), wobei jeder erste flexible elektrische Leiter elektrisch mit einem ersten elektrischen Kontakt (110-122) verbunden ist,
einen Kontaktabschnitt, der eine Vielzahl von zweiten elektrischen Kontakten umfasst, wobei jeder zweite elektrische Kontakt elektrisch mit einem ersten elektrischen Leiter (150-162) verbunden ist und ferner elektrisch mit einem zweiten elektrischen Leiter oder einem Wandleranschluss eines Ultraschallverarbeitungsgeräts verbunden oder verbindbar ist, und
**dadurch gekennzeichnet, dass** der flexible Basisabschnitt und die Vielzahl von ersten flexiblen elektrischen Leitern eine einzige flexible gedruckte Schaltung sind, und wobei sich die ersten flexiblen elektrischen Leiter (150-162) von dem flexiblen Basisabschnitt (103) und in den Griff erstrecken.

2. Endoskop nach Anspruch 1, wobei sich die Vielzahl von ersten flexiblen elektrischen Leitern (150-162) für einen ersten Teil ihrer Länge innerhalb des Einführrohrs erstreckt, sich für einen zweiten Teil ihrer Länge innerhalb des Griffs erstreckt und sich für einen dritten Teil ihrer Länge innerhalb des ersten flexiblen Kabels erstreckt.

3. Endoskop nach Anspruch 2, wobei das erste flexible Kabel einen Ultraschallverbinder umfasst, der in einen Wandleranschluss eines Ultraschallverarbeitungsgeräts einführbar ist, um elektrische Signale, die durch das Ultraschallwandler-Array generiert werden, an das Ultraschallverarbeitungsgerät bereitzustellen, und elektrische Signale zu empfangen, die durch das Ultraschallverarbeitungsgerät generiert werden, wobei die Vielzahl von ersten flexiblen elektrischen Leitern (150-162) sich über die gesamte Länge des ersten flexiblen Kabels erstreckt und der Ultraschallverbinder der Kontaktabschnitt ist.

4. Endoskop nach Anspruch 3, wobei das Endoskop ferner eine Bildaufnahmevorrichtung umfasst, und wobei das erste flexible Kabel ferner dazu ausgelegt ist, elektrische Signale von der Bildaufnahmevorrichtung zu empfangen.

5. Endoskop nach einem der Ansprüche 1 bis 4, wobei die Vielzahl von ersten flexiblen elektrischen Leitern (150-162) mindestens einmal gefaltet ist, um ihre Länge zu vergrößern.

6. Endoskop nach einem der Ansprüche 1 bis 5, wobei die obere Oberfläche (102) eine konvexe obere Oberfläche ist, die Vielzahl von ersten flexiblen elektrischen Leitern (150-162) an einem ersten Kurvenabschnitt (140) und an einem zweiten Kurvenabschnitt (141) um die Stützstruktur (101) gebogen ist, wobei der erste Kurvenabschnitt (140) separat von dem zweiten Kurvenabschnitt (141) vorliegt.

7. Endoskop nach Anspruch 6, wobei die Stützstruktur (101) eine erste Seitenoberfläche (104) aufweist, die in einer Ebene angeordnet ist, die rechtwinklig zu der ersten Achse (191) ist, wobei die erste Seitenoberfläche (104) mit der konvexen oberen Oberfläche (102) verbunden ist, und wobei der erste Kurvenabschnitt (140) entlang der Verbindung zwischen der konvexen oberen Oberfläche (102) und der ersten Seitenoberfläche (104) angeordnet ist.

8. Endoskop nach Anspruch 7, wobei der zweite Kurvenabschnitt (141) entlang der Verbindung zwischen der konvexen oberen Oberfläche und der ersten Seitenoberfläche (104) angeordnet und durch einen Spalt (142) von dem ersten Kurvenabschnitt (140) abgeteilt ist.

9. Endoskop nach Anspruch 8, wobei die Stützstruktur eine konkave untere Oberfläche (106) gegenüber der konvexen oberen Oberfläche (102) umfasst, die erste Seitenoberfläche (104) mit der konkaven unteren Oberfläche (106) verbunden ist, der Spalt (142) sich entlang der gesamten ersten Seite (104) der Stützstruktur (101) erstreckt, und wobei die Vielzahl der ersten flexiblen elektrischen Leiter (150-162) ferner an einem dritten Kurvenabschnitt (143) und an einem vierten Kurvenabschnitt (144) um die Stützstruktur gebogen ist, wobei der dritte Kurvenabschnitt (143) durch den Spalt (142) separat von dem vierten Kurvenabschnitt (144) vorliegt, und wobei der dritte Kurvenabschnitt (143) und der vierte Kurvenabschnitt (144) entlang der Verbindung zwischen der ersten Seitenoberfläche (104) und der konkaven unteren Oberfläche (106) angeordnet sind.

10. Gedruckte flexible elektrische Schaltung zum Empfangen und Bereitstellen elektrischer Signale an ein Ultraschallwandler-Array, umfassend
eine Vielzahl von Ultraschallwandlerelementen, wobei die gedruckte flexible elektrische Schaltung Teil eines Endoskops nach Anspruch 1 bildet und Folgendes umfasst
einen flexiblen Basisabschnitt (103), der eine Vielzahl von ersten elektrischen Kontakten (110-122) umfasst, wobei jeder erste elektrische Kontakt (110-122) mit einem Ultraschallwandlerelement zum Bereitstellen und Empfangen elektrischer Signale an das Ultraschallwandlerelement verbindbar ist und sich entlang einer Mittelachse (193) erstreckt, die parallel zu einer ersten Achse (191) ist,
eine Vielzahl von ersten flexiblen elektrischen Leitern (150-162), wobei jeder erste flexible elektrische Leiter elektrisch mit einem ersten elektrischen Kontakt (110-122) verbunden ist, und
wobei die Vielzahl von ersten flexiblen elektrischen Leitern (150-162) mit einem Ultraschallverbinder verbindbar ist, wodurch die ersten flexiblen elektrischen Leiter für die gesamte elektrische Kopplung zwischen dem Ultraschallverbinder und dem Ultraschallwandler-Array verantwortlich sein können.

11. Gedruckte flexible elektrische Schaltung, wobei die Vielzahl von ersten flexiblen elektrischen Leitern (150-162) eine Länge von mindestens 40 cm aufweist.

12. System, umfassend ein Endoskop nach einem der Ansprüche 1 bis 9 und ein Ultraschallverarbeitungsgerät, wobei das Endoskop eine Bildaufnahmevorrichtung umfasst und das erste flexible Kabel mit dem Ultraschallverarbeitungsgerät verbindbar ist.

13. System nach Anspruch 12, wobei das erste flexible Kabel ferner dazu ausgelegt ist, elektrische Signale von der Bildaufnahmevorrichtung zu empfangen und die empfangenen elektrischen Signale an das Ultraschallverarbeitungsgerät bereitzustellen, und wobei das Ultraschallverarbeitungsgerät eine oder mehrere Verarbeitungseinheiten umfasst, die dazu ausgelegt sind, sowohl die von dem Ultraschallwandler empfangenen Signale als auch die von der Bildaufnahmevorrichtung empfangenen Signale zu verarbeiten.

14. System nach Anspruch 13, wobei das System ferner ein Videoverarbeitungsgerät umfasst, und wobei das Endoskop ferner ein zweites flexibles Kabel umfasst, das dazu ausgelegt ist, elektrische Signale von der Bildaufnahmevorrichtung zu empfangen und die empfangenen elektrischen Signale an das Videoverarbeitungsgerät bereitzustellen.

## Revendications

1. Endoscope comprenant une poignée, un tube d'insertion ayant une extrémité proximale et une extrémité distale, un premier câble flexible et un transducteur ultrasonore pour recevoir et transmettre des ultrasons, le transducteur ultrasonore comprenant un réseau de transducteur ultrasonore fixé à l'extrémité distale du tube d'insertion, la poignée étant fixée à l'extrémité proximale du tube d'insertion, le premier câble flexible s'étendant depuis la poignée et étant connecté électriquement au réseau de transducteur ultrasonore et pouvant être connecté à un appareil de traitement ultrasonore et configuré pour fournir à l'appareil de traitement ultrasonore des signaux électriques générés par le réseau de transducteur ultrasonore et recevoir des signaux électriques générés par l'appareil de traitement ultrasonore pour le réseau de transducteur ultrasonore, et dans lequel le réseau de transducteur ultrasonore comprend une pluralité d'éléments de transducteur ultrasonore (180), et dans lequel le transducteur ultrasonore comprend un circuit électrique destiné à recevoir et à fournir des signaux électriques à la pluralité d'éléments de transducteur ultrasonore (180), et une structure de support (101) présentant une surface supérieure (102) faisant face à la pluralité d'éléments de transducteur ultrasonore (180) et s'étendant dans une direction parallèle à un premier axe (191), dans lequel le circuit électrique comprend
une section de base flexible (103) comprenant une pluralité de premiers contacts électriques (110 à 122), chaque premier contact électrique (110 à 122) étant connecté à un élément de transducteur ultrasonore pour fournir et recevoir des signaux électriques à/de l'élément de transducteur ultrasonore et s'étendant le long d'un axe central (193) parallèle au premier axe (191), la section de base flexible (103) étant disposée sur la surface supérieure (102) de la structure de support (101),
une pluralité de premiers conducteurs électriques flexibles (150 à 162), chaque premier conducteur électrique flexible étant connecté électriquement à un premier contact électrique (110 à 122),
une section de contact comprenant une pluralité de seconds contacts électriques, chaque second contact électrique étant électriquement connecté à un premier conducteur électrique (150 à 162) et en outre électriquement connecté ou pouvant l'être à un second conducteur électrique ou à un port de transducteur d'un appareil de traitement ultrasonore, et
**caractérisé en ce que** la section de base flexible et la pluralité de premiers conducteurs électriques flexibles sont un seul circuit imprimé flexible et dans lequel les premiers conducteurs électriques flexibles (150 à 162) s'étendent à partir de la section de base flexible (103) et dans la poignée.

2. Endoscope selon la revendication 1, dans lequel la pluralité de premiers conducteurs électriques flexibles (150 à 162) pour une première partie de sa longueur s'étend à l'intérieur du tube d'insertion, pour une deuxième partie de sa longueur s'étend à l'intérieur de la poignée, et pour une troisième partie de sa longueur s'étend à l'intérieur du premier câble flexible.

3. Endoscope selon la revendication 2, dans lequel le premier câble flexible comprend un connecteur ultrasonore pouvant être inséré dans un orifice de transducteur d'un appareil de traitement ultrasonore pour fournir des signaux électriques générés par le réseau de transducteur ultrasonore à l'appareil de traitement ultrasonore et recevoir des signaux électriques générés par l'appareil de traitement ultrasonore, dans lequel la pluralité de premiers conducteurs électriques flexibles (150 à 162) s'étend sur toute la longueur du premier câble flexible et le connecteur ultrasonore est la section de contact.

4. Endoscope selon la revendication 3, l'endoscope comprenant en outre un dispositif de capture d'image et dans lequel le premier câble flexible est en outre configuré pour recevoir des signaux électriques du dispositif de capture d'image.

5. Endoscope selon l'une quelconque des revendications 1 à 4, dans lequel les conducteurs de la pluralité de premiers conducteurs électriques flexibles (150 à 162) sont pliés au moins une fois pour étendre leur longueur.

6. Endoscope selon l'une quelconque des revendications 1 à 5, dans lequel la surface supérieure (102) est une surface supérieure convexe, les conducteurs de la pluralité de premiers conducteurs électriques flexibles (150 à 162) sont courbés autour de la structure de support (101) au niveau d'une première section courbe (140) et au niveau d'une deuxième section courbe (141), la première section courbe (140) étant séparée de la deuxième section courbe (141).

7. Endoscope selon la revendication 6, dans lequel la structure de support (101) a une première surface latérale (104) agencée dans un plan perpendiculaire au premier axe (191), la première surface latérale (104) étant reliée à la surface supérieure convexe (102) et dans lequel la première section courbe (140) est agencée le long de la liaison entre la surface supérieure convexe (102) et la première surface latérale (104).

8. Endoscope selon la revendication 7, dans lequel la deuxième section courbe (141) est agencée le long de la liaison entre la surface supérieure convexe et la première surface latérale (104) et éloignée de la première section courbe (140) par un espace (142).

9. Endoscope selon la revendication 8, dans lequel la structure de support comprend une surface inférieure concave (106) opposée à la surface supérieure convexe (102), la première surface latérale (104) est reliée à la surface inférieure concave (106), l'espace (142) s'étendant le long de tout le premier côté (104) de la structure de support (101), et dans lequel les conducteurs de la pluralité des premiers conducteurs électriques flexibles (150 à 162) sont en outre courbés autour de la structure de support au niveau d'une troisième section courbe (143) et au niveau d'une quatrième section courbe (144), la troisième section courbe (143) étant séparée de la quatrième section courbe (144) par l'espace (142), et dans lequel la troisième section courbe (143) et la quatrième section courbe (144) sont agencées le long de la liaison entre la première surface latérale (104) et la surface inférieure concave (106).

10. Circuit imprimé électrique flexible destiné à recevoir et à fournir des signaux électriques à un réseau de transducteur ultrasonore comprenant
une pluralité d'éléments de transducteur ultrasonore, le circuit imprimé électrique flexible faisant partie d'un endoscope selon la revendication 1 et comprenant
une section de base flexible (103) comprenant une pluralité de premiers contacts électriques (110 à 122), chaque premier contact électrique (110 à 122) pouvant être connecté à un élément de transducteur ultrasonore pour fournir et recevoir des signaux électriques à/de l'élément de transducteur ultrasonore et s'étendant le long d'un axe central (193) parallèle à un premier axe (191),
une pluralité de premiers conducteurs électriques flexibles (150 à 162), chaque premier conducteur électrique flexible étant connecté électriquement à un premier contact électrique (110 à 122), et
dans lequel la pluralité de premiers conducteurs électriques flexibles (150 à 162) peut être connectée à un connecteur ultrasonore, moyennant quoi les premiers conducteurs électriques flexibles peuvent être responsables de la totalité du couplage électrique entre le connecteur ultrasonore et le réseau de transducteur ultrasonore.

11. Circuit imprimé électrique flexible dans lequel les conducteurs de la pluralité de premiers conducteurs électriques flexibles (150 à 162) ont une longueur d'au moins 40 cm.

12. Système comprenant un endoscope selon l'une quelconque des revendications 1 à 9 et un appareil de traitement ultrasonore, dans lequel l'endoscope comprend un dispositif de capture d'image et le premier câble flexible peut être connecté à l'appareil de traitement ultrasonore.

13. Système selon la revendication 12, dans lequel le premier câble flexible est en outre configuré pour recevoir des signaux électriques du dispositif de capture d'image et fournir les signaux électriques reçus à l'appareil de traitement ultrasonore, et dans lequel l'appareil de traitement ultrasonore comprend une ou plusieurs unités de traitement configurées pour traiter à la fois les signaux reçus du transducteur ultrasonore et les signaux reçus du dispositif de capture d'image.

14. Système selon la revendication 13, le système comprenant en outre un appareil de traitement vidéo et dans lequel l'endoscope comprend en outre un second câble flexible configuré pour recevoir des signaux électriques du dispositif de capture d'image et fournir les signaux électriques reçus à l'appareil de traitement vidéo.
